(19) Europäisches Patentamt
European Patent Office
Office européen des brevets

(11) EP 0 880 496 B1

(12) FASCICULE DE BREVET EUROPEEN

(45) Date de publication et mention de la délivrance du brevet: **29.05.2002 Bulletin 2002/22**

(21) Numéro de dépôt: **97902408.0**

(22) Date de dépôt: **30.01.1997**

(51) Int Cl.[7]: **C07C 217/20**, C07C 39/15, C07C 39/38, A61K 31/05, A61K 31/055, C07C 49/753, C07C 43/23

(86) Numéro de dépôt international: **PCT/FR97/00184**

(87) Numéro de publication internationale: **WO 97/28116 (07.08.1997 Gazette 1997/34)**

(54) **COMPOSES BIPHENYLES ET LEUR UTILISATION COMME AGENTS ESTROGENIQUES**
BIPHENYLVERBINDUNGEN SOWIE IHRE ANWENDUNG ALS ÖSTROGENE MITTEL
BIPHENYL COMPOUNDS AND USE THEREOF AS OESTROGENIC AGENTS

(84) Etats contractants désignés: **AT BE CH DE DK ES FI FR GB GR IE IT LI LU NL PT SE**

(30) Priorité: **01.02.1996 FR 9601212**

(43) Date de publication de la demande: **02.12.1998 Bulletin 1998/49**

(73) Titulaire: **Aventis Pharma S.A. 92160 Antony (FR)**

(72) Inventeur: **LESUISSE, Dominique F-75018 Paris (FR)**

(56) Documents cités:

- **PATENT ABSTRACTS OF JAPAN vol. 017, no. 327 (C-1073), 22 Juin 1993 & JP 05 032579 A (BANYU PHARMACEUTCAL), 9 Février 1993,**
- **HELVETICA CHIMICA ACTA, vol. 58, no. 1, 29 Janvier 1975, BASEL, CH, pages 104-110, XP002016375 C. EGLI, ET AL.: "Die Benzoin-Phenol-Synthese"**
- **JOURNAL OF ORGANIC CHEMISTRY, vol. 14, no. 1, Janvier 1949, WASHINGTON, DC, US, pages 163-168, XP002016371 C.F.H. ALLEN, ET AL.: "The chemistry of o-terphenyl. III. Sulphonic acids"**
- **AUSTRALIAN JOURNAL OF CHEMISTRY, vol. 9, 1956, MELBOURNE, AU, pages 373-381, XP000605749 J. CYMERMAN-CRAIG, ET AL.: "The condensation of acetylenes with deoxyanisoin"**
- **PROCEEDINGS OF THE ROYAL SOCIETY OF LONDON, SERIES B - BIOLOGICAL SCIENCES, vol. 140, 1953, LONDON, GB, pages 470-497, XP000605977 E.C. DODDS, ET AL.: "Synthetic oestrogenic compounds related to stilbene and diphenylethane. III"**
- **HOPPE-SEYLER'S ZEITSCHRIFT FÜR PHYSIOLOGISCHE CHEMIE, vol. 274, 1942, BERLIN, DE, pages 39-47, XP000605795 W. SALZER: "Über neue synthetische, hochwirsame Östrogene"**
- **JOURNAL OF THE AMERICAN CHEMICAL SOCIETY, vol. 55, no. 7, Juillet 1933, WASHINGTON, DC, US, page 3048 XP002016372 S.S. JENKINS: "The synthesis of isomeric unsymmetrical benzoins"**
- **JOURNAL OF THE AMERICAN CHEMICAL SOCIETY, vol. 52, no. 10, Octobre 1930, WASHINGTON, DC, US, pages 4107-4109, XP002016374 J.S BUCK, ET AL.: "Mixed benzoins. II"**
- **JOURNAL OF THE AMERICAN CHEMICAL SOCIETY, vol. 86, no. 4, 20 Février 1964, WASHINGTON, DC, US, pages 684-687, XP002016373 K. LEROI NELSON, ET AL.: "The mechanistic fate of the anilino moiety in the rearrangement of alpha-anilinoketones"**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 880 496 B1

## Description

L'invention a pour objet de nouveaux composés biphényles, leur procédé de préparation et les intermédiaires de ce procédé, leur application à titre de médicaments et les compositions pharmaceutiques les renfermant.

F.Knoop et al. (Hoppe-Seyler's Zeitschrift fur Physiologische Chemie), décrit des dérivés de dihydronaphtalènes pouvant présenter une affinité pour le récepteur Estrogène.

JC Craig et al (Australian J. Of Chem vol 9 (1956) p. 373-381) décrit le procédé de condensation d'acétylènes avec des déoxyanisoines dans le but d'obtenir des dérivés de napthtalènes ayant des propriétés estrogènes.

L'invention a pour objet les composés de formule générale (I) :

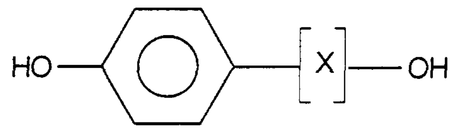

(I)

dans laquelle [X] représente les carbocycles aromatiques suivants :

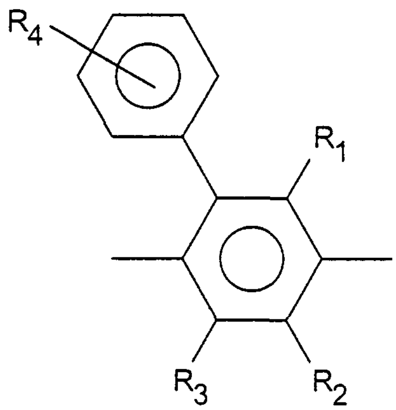

(A)

ou

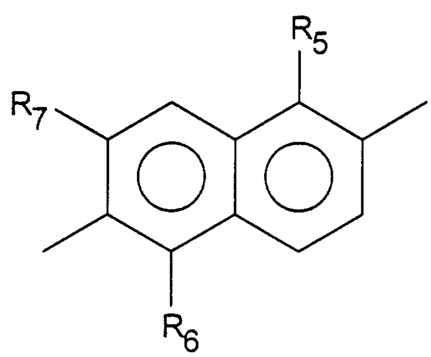

(B)

dans lesquels $R_1$ représente un radical alkyle renfermant de 1 à 4 atomes de carbone ou un atome d'hydrogène, $R_2$ représente un radical alkyle renfermant de 1 à 4 atomes de carbone ou un atome d'hydrogène, $R_3$ représente un atome d'hydrogène, un atome d'halogène, un radical alkyle renfermant de 1 à 4 atomes de carbone ou un radical alkoxy renfermant de 1 à 4 atomes de carbone, $R_4$ en position para ou méta représente un atome d'hydrogène, un atome d'halogène, un radical hydroxyle, un radical alkyle, alkényle ou alkynyle renfermant au plus 4 atomes de carbone, un radical alkoxy, alkylthio dans lesquels alkyle renferme de 1 à 4 atomes de carbone, un groupement - $NR_AR_B$ dans lequel $R_A$ et $R_B$ identiques ou différents représentent un atome d'hydrogène, un radical alkyle renfermant de 1 à 4 atomes de carbone ou forment ensemble avec l'azote auquel ils sont liés un hétérocycle saturé à 5 ou 6 chaînons renfermant éventuellement un second hétéroatome choisi parmi l'azote, l'oxygène et le soufre, ce groupement - $NR_AR_B$ étant éventuellement sous forme oxydée, un groupement de formule générale $-O-(CH_2)_n-NR_AR_B$ dans laquelle n est

un entier qui varie de 2 à 7 et dans laquelle $-NR_AR_B$ est tel que défini précédemment, $R_5$ représente un atome d'hydrogène ou un atome d'halogène, $R_6$ et $R_7$ identiques ou différents représentent un atome d'hydrogène, un atome d'halogène, un radical alkyle renfermant de 1 à 4 atomes de carbone, ou un radical phényle éventuellement substitué en position méta ou para par un radical $R_4$ tel que défini précédemment ainsi que les sels d'addition avec les acides ou les bases, à l'exception des composés de formule (I) dans laquelle [X] représente le groupe (A) dans lequel $R_1$, $R_2$, $R_3$ sont des atomes d'hydrogène et $R_4$ représente un radical hydroxyle et ceux dans laquelle [X] représente le groupe (B) dans lequel $R_5$, $R_6$ et $R_7$ sont des atomes d'hydrogène ou bien $R_5$ et $R_6$ sont des atomes d'hydrogène et $R_7$ représente un radical alkyle renfermant de 1 à 4 atomes de carbone.

Lorsque $R_1$, $R_2$, $R_3$, $R_4$, $R_6$, $R_7$, $R_A$ et $R_B$ représentent un radical alkyle renfermant de 1 à 4 atomes de carbone, il s'agit du radical méthyle, éthyle, propyle, isopropyle, butyle, isobutyle ou tert-butyle. Lorsque $R_3$, $R_4$, $R_5$, $R_6$ et $R_7$ sont des atomes d'halogène, il s'agit du fluor, du chlore, du brome ou de l'iode. Il s'agit, de préférence, du chlore. Lorsque $R_4$ est un radical alkényle renfermant au plus 4 atomes de carbone, il s'agit de préférence du radical vinyle ou propényle. Lorsque $R_4$ est un radical alkynyle renfermant au plus 4 atomes de carbone, il s'agit de préférence du radical éthynyle ou propynyle. Lorsque $R_3$ ou $R_4$ représentent un radical alkyloxy renfermant de 1 à 4 atomes de carbone, il s'agit de préférence du radical méthoxy, éthoxy, propyloxy, isopropyloxy ou butyloxy. Lorsque $R_4$ est un radical alkylthio renfermant de 1 à 4 atomes de carbone, il s'agit de préférence du radical méthylthio, éthylthio, propylthio, isopropylthio ou butylthio. Lorsque $R_4$ est un radical $NR_AR_B$ dans lequel $R_A$ et $R_B$ identiques ou différents représentent un atome d'hydrogène ou un radical alkyle renfermant de 1 à 4 atomes de carbone, il s'agit de préférence du radical amino, méthylamino, éthylamino, diméthylamino, diéthylamino ou méthyléthylamino. Lorsque $R_4$ est un groupement $-NR_AR_B$ dans lequel $R_A$ et $R_B$ forment avec l'azote un hétérocycle saturé, il s'agit de préférence des groupements pyrrolidino, pipéridino, pipérazino, morpholino ou thiomorpholino, chacun de ces groupements amino étant éventuellement sous forme oxydée.

L'invention s'étend naturellement aux sels des composés de formule (I), notamment lorsque les composés de formule (I) comportent une fonction amino. Il s'agit des sels formés par exemple avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcanesulfoniques tels que les acides méthane et éthanesulfoniques, arène-sulfoniques, tels que les acides benzène et paratoluène sulfoniques et arylcarboxyliques.

Il s'agit également des sels formés sous l'action d'une base ou d'un métal alcalin ou alcalinoterreux, afin d'obtenir par exemple des dérivés tels que l'alcoolate de sodium ou de potassium ou des dérivés tels que le phénolate de potassium ou de sodium.

L'invention a plus particulièrement pour objet les composés de formule générale (I) telle que définie précédemment dans laquelle [X] est le carbocycle aromatique de formule générale (A).

L'invention a plus particulièrement pour objet les produits de formule générale (I) telle que définie précédemment dans laquelle [X] est le carbocycle aromatique de formule générale (B).

L'invention a plus particulièrement pour objet les produits de formule générale (I) telle que définie plus haut, répondant à la formule générale (I') :

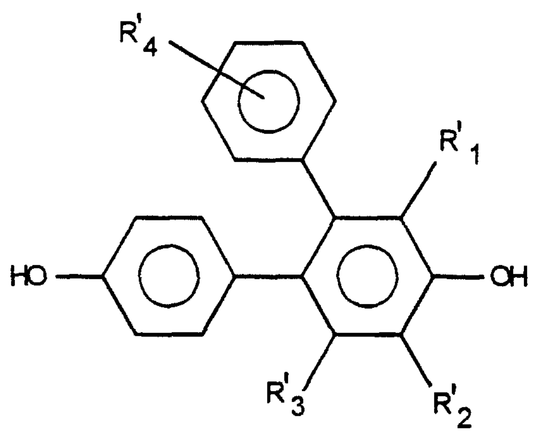

(I')

dans laquelle $R'_1$, $R'_2$ et $R'_3$ représentent un atome d'hydrogène ou un radical alkyle renfermant de 1 à 4 atomes de carbone, $R'_4$ en position méta ou para représente un atome d'hydrogène, un atome d'halogène, un radical hydroxyle, un radical alkyle, un groupement $-NR_AR_B$ ou un groupement $-O-(CH_2)_n-NR_AR_B$, n, $R_A$ et $R_B$ étant tels que définis précédemment, ainsi que les sels d'addition avec les acides. Lorsque $R'_4$ est un groupement $-O-(CH_2)_nNR_AR_B$, il s'agit de préférence du groupement $-O-(CH_2)_2-NMe_2$.

L'invention a plus particulièrement pour objet les produits de formule générale (I) telle que définie précédemment répondant à la formule générale (I') dans laquelle $R'_1$, $R'_2$ et $R'_3$ sont des atomes d'hydrogène.
L'invention a tout spécialement pour objet le composé de formule générale (I) telle que définie précédemment dans laquelle $R_6$ représente un atome d'halogène ou un groupement

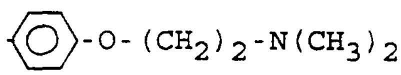

et $R_7$ représente un atome d'hydrogène.
L'invention a tout spécialement pour objet le composé de formule générale (I) telle que définie plus haut dont les noms suivent :

- 5-[4-[2-(diméthylamino) éthoxy] phényl] 6-(4-hydroxyphényl) 2-naphtalénol,
- 1,5-dichloro-6-(4-hydroxyphényl)-2-naphtalénol,
- 5-chloro-6-(4-hydroxyphényl)-2-naphtalénol.

L'invention a également pour objet un procédé de préparation des produits de formule (I) telle que définie ci-dessus caractérisé en ce que l'on soumet un produit de formule (II) :

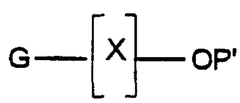

(II)

dans laquelle [X] est tel que défini précédemment, P' représente un groupement protecteur, et G représente un atome d'halogène ou un groupement $OSO_2CF_3$ à l'action, en présence d'un catalyseur, d'un produit de formule (III) :

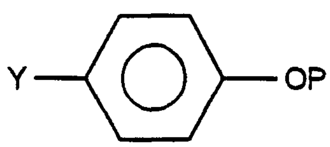

(III)

dans laquelle Y représente un atome d'halogène, un groupement $B(OH)_2$ ou un groupement $Sn(R)_3$, dans lequel R représente un groupement alkyle renfermant de 1 à 8 atomes de carbone et P représente un groupement protecteur identique ou différent de P', pour obtenir un produit de formule (IV) :

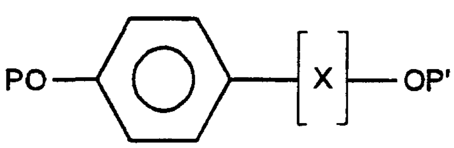

(IV)

dans laquelle P, P' et [X] ont la même signification que précédemment, produit de formule (IV) que l'on soumet à une ou plusieurs réactions de déprotection afin d'obtenir le produit de formule (I) tel que défini précédemment que, le cas échéant l'on soumet à l'action d'un acide ou d'une base pour obtenir le sel correspondant.
La formation des biphényles de formule (IV) par couplage du composé aromatique de formule (II) avec le composé aromatique de formule (III) s'effectue en présence d'un catalyseur choisi parmi les dérivés du palladium ou en présence de cuivre dans le cas où Y est un atome d'iode et ainsi peut s'effectuer dans les conditions décrites dans les articles suivants :

- A. Huth, I. Beetz and I. Schumann Tetrahedron (1989) 45 6679 : Conditions : $Na_2CO_3$ 2M/$Pd(P\Phi_3)_4$/Toluène/LiCl/EtOH/Δ
- J. K. Stille Ang. Chem. Int. Ed. (1986) 25 508 : Conditions : $Pd(P\Phi_3)_4$/LiCl/Dioxane/Δ

- T. Oh-e, N. Migawa and A. Suzuki J. Org. Chem. (1993) 58 2201-2208 : Conditions : $K_3PO_4$/KBr/Pd($P\Phi_3$)$_4$/Dioxane/Δ
- P. E. Fank Chem. Rev. (1964) 38 139 : Conditions : Cu/DMF/120°C dans le cas où Y est un atome d'iode.
- E. Erdik Tetrahedron (1992) 48 9577 : Conditions : nBuLi/THF/-78°C - 2) ZnCl2 - 3) ArBr/Pd($P\Phi_3$)$_4$/Δ 4)Hcl/MeOH.

L'invention a également pour objet un procédé de préparation des produits de formule (I) dans laquelle [X] est le carbocycle aromatique de formule (A) telle que définie ci-dessus caractérisé en ce que l'on soumet un produit de formule (V) :

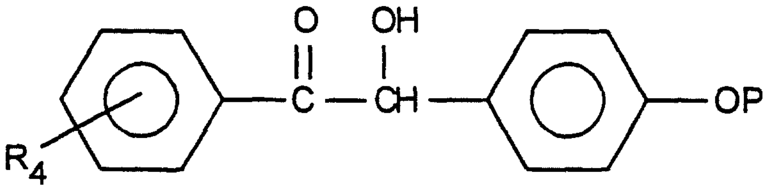

(V)

dans laquelle $R_4$ et P sont tels que définis précédemment, à l'action de la méthylvinylcétone de formule générale (VI) :

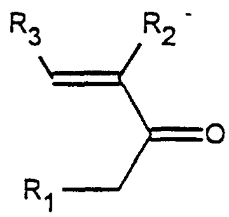

(VI)

dans laquelle $R_1$, $R_2$ et $R_3$ sont tels que définis précédemment, pour obtenir le produit de formule (VII) :

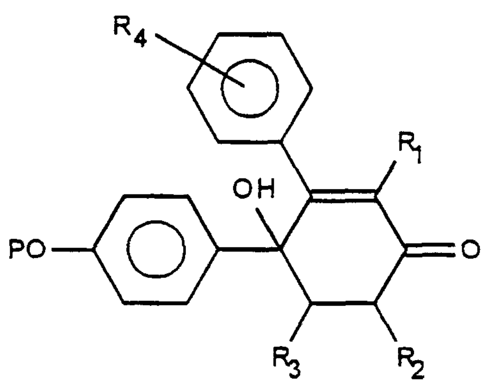

(VII)

dans laquelle $R_1$, $R_2$, $R_3$, $R_4$ et P sont tels que définis précédemment,
que l'on soumet à l'action d'un réactif de déshydratation et d'aromatisation afin d'obtenir le produit de formule (VIII) :

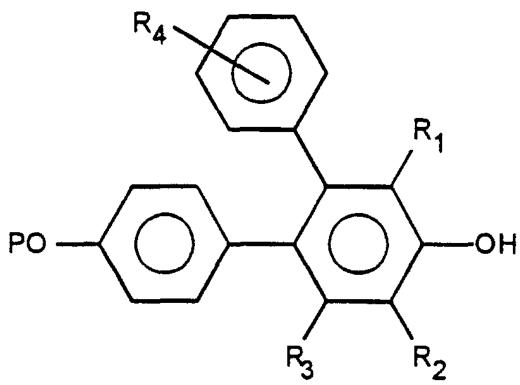

(VIII)

dans laquelle $R_1$, $R_2$, $R_3$, $R_4$ et P sont tels que définis précédemment, que l'on soumet à l'action d'un réactif de déprotection afin d'obtenir les produits de formule (I) dans laquelle [X] est le carbocycle aromatique de formule (A) que si désiré, l'on soumet à l'action d'un acide pour obtenir un sel correspondant.

Les groupements protecteurs P ou P' sont de préférence choisis parmi un radical alkyle renfermant de 1 à 4 atomes de carbone, un groupement benzyle et un groupement $R_CR_DR_ESi$, dans lequel $R_C$, $R_D$ et $R_E$ identiques ou différents représentent un radical alkyle renfermant de 1 à 4 atomes de carbone ou un groupement phényle. Il s'agira tout particulièrement des radicaux méthyle, phényle, terbutyldiméthylsilyle et terbutyldiphénylsilyle.

L'action de la méthylvinylcétone de formule générale (III) sur le produit de formule (II) s'effectue préférentiellement en présence d'une base telle que la potasse dans un mélange dioxane/eau.

La réaction de déshydratation et d'aromatisation s'effectue par exemple à l'aide d'un acide minéral tel que l'acide phosphorique à une température de 150°C pendant 4 heures.

Les réactions de déprotection sont les méthodes de déprotection classiques connues de l'homme du métier. Une liste assez complète se trouve dans l'ouvrage suivant : Protective groups in organic synthesis T.W greene, John Wiley & sons (1981).

A titre d'exemple, lorsque P ou P' représentent un radical méthyle la réaction de déprotection peut s'effectuer par action de tribromoborane dans le dichlorométhane ou d'acide chlorhydrique dans la pyridine. Lorsque P ou P' représentent un groupement benzyle on peut réaliser une hydrogénation catalytique ou une hydrolyse à l'acide trifluoroacétique. Lorsque P ou P' représentent un groupement silylé la déprotection peut s'effectuer par le fluorure de tétrabutylammonium (TBAF) dans le tetrahydrofuranne (THF).

La salification par un acide ou une base est réalisée dans des conditions usuelles. On opère par exemple avec de l'acide chlorhydrique, en solution éthérée.

Les composés de formule (I) ainsi que leurs sels d'addition avec les acides ou les bases pharmaceutiquement acceptables sont des produits particulièrement intéressants du point de vue pharmacologique.

Ce sont des ligands originaux du récepteur estrogène. A ce titre, ils peuvent être utilisés dans le traitement des troubles liés à une hypofolliculinie, par exemple, les aménorrhées, les dysménorrées, les avortements répétés, les troubles prémenstruels, dans le traitement de certaines pathologies estrogéno-dépendantes telles que les adénomes ou carcinomes prostatiques, les carcinomes mammaires et ses métastases ou dans le traitement des tumeurs bénignes du sein, en tant qu'antiutérotrophique ainsi que dans le traitement substitutif des symptômes liés à la ménopause et en particulier de l'ostéoporose.

L'invention a donc pour objet à titre de médicaments les produits de formule (I) telle que décrite précédemment ainsi que leurs sels d'addition avec les acides ou les bases pharmaceutiquement acceptables.

L'invention a plus particulièrement pour objet à titre de médicaments les composés de formule (I) telles que décrite précédemment répondant à la formule générale (I') telle que décrite précédemment ainsi que les sels d'addition avec les acides ou les bases pharmaceutiquement acceptables.

L'invention a tout particulièrement pour objet à titre de médicaments les produits de formule (I) suivants :

le 5-[4-[2-(diméthylamino) éthoxy] phényl] 6-(4-hydroxyphényl) 2-naphtalénol,
le 5-chloro-6-(4-hydroxyphényl)2-naphtalénol,
le 1,5-dichloro-6-(4-hydroxyphényl)-2-naphtalénol.

L'invention s'étend aux compositions pharmaceutiques renfermant comme principe actif au moins un médicament tel que défini ci-dessus.

Les composés de formule (I) sont utilisés par voie digestive, parentérale ou locale, par exemple par voie percutanée. Ils peuvent être prescrits sous forme de comprimés simples ou dragéifiés, de gélules, de granulés, de

suppositoires, d'ovules, de préparation injectables, de pommades, de crèmes, de gels, de microsphères, d'implants, de patchs, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie varie en fonction de l'affection à traiter et de la voie d'administration : elle peut varier par exemple de 1 mg à 100 mg par jour chez l'adulte par voie orale.

Les produits de formule générale (V) telle que définie plus haut sont obtenus par action du produit de formule générale (IX) :

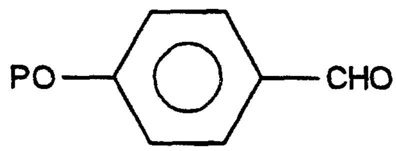

(IX)

avec un produit de formule générale (X) :

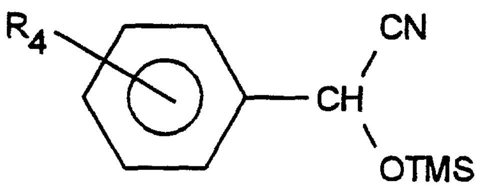

(X)

en présence d'une base forte telle que la LDA (diisopropylamide lithium).
Cette réaction est décrite dans :
S. HUNIG et al. Chem. Ber. (1980) 113, 324-332

Les produits de formule (X) sont obtenus par action du cyanure de triméthyl silyle en présence d'un acide de Lewis tel que $ZnI_2$, sur l'aldéhyde correspondant de formule générale (XI) :

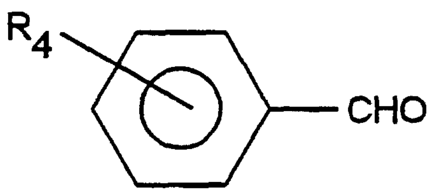

(XI)

Cette réaction est décrite dans Synthesis (1980) p. 861-868. Les produits protégés de formule (IX) sont obtenus à partir du parahydroxy benzaldéhyde par une des méthodes classiques de protection des alcools décrites dans l'ouvrage de T.W. Greene cité plus haut.

Les produits de formules (II), (III), (IX), (X) et (XI) sont des produits commerciaux ou aisément accessibles par des méthodes classiques de fonctionnalisation des composés aromatiques connues par l'homme du métier.

Les produits de formule (VI) sont également aisément accessibles à l'homme du métier.

Les produits de formule (V) dans lesquels $R_4$ est un alkyloxy renfermant de 1 à 4 atomes de carbone en position para et P est un alkyle renfermant de 1 à 4 atomes de carbone, sont connus et sont décrits dans les références suivantes :

Chemical Abstract : 65-10442b, 112-148858n, 59-9865f.

L'invention a enfin pour objet à titre de produits industriels nouveaux et notamment à titre de produits intermédiaires nouveaux nécessaires à la mise en oeuvre de l'invention, les produits de formule générale (IV), (VII) et (VIII) telles que définies précédemment, à l'exception des produits de formule (VII) et (VIII) dans lesquels $R_4$ est un radical méthoxy, P est un radical méthyle et $R_1$, $R_2$ et $R_3$ sont des atomes d'hydrogène, et à l'exception des produits de formule (IV) dans laquelle P et P' sont des radicaux méthyle ou acyle, X représente le groupe B dans lequel $R_5$, $R_6$ et $R_7$ sont des atomes d'hydrogène.

Les exemples suivants illustrent l'invention sans toutefois la limiter :

**<u>PREPARATION 1</u> : Acide-[4-(phénylméthoxy)phényl]-boronique**

<u>Stade A</u> : 1-bromo-4-(phénylméthoxy)-benzène

On ajoute, à 0°C, 15,26 g d'hydrure de sodium à 50 % dans l'huile à une solution sous gaz inerte de 50 g de parabromophénol dans 320 ml de diméthylformamide (DMF), agite pendant 30 minute à 0°C, puis ajoute 37,7 ml de bromure-de benzyle. On agite pendant 2 heures 30 en laissant remonter la température à 20°C, puis coule le mélange réactionnel sur de l'eau glacée, filtre le précipité, et sèche. On obtient 73,35 g de produit attendu. Rf : 0,85 (chromatographie sur couche mince, support : silice, éluant : cyclohexane/acétate d'éthyle 7/3).
Spectre I.R. : ($CHCl_3$)
Absence d'OH
Aromatique 1592, 1580 et 1488 $cm^{-1}$

<u>Stade B</u> : Acide [4-(phénylméthoxy)phényl]-boronique

On ajoute, goutte à goutte, sous gaz inerte et à -78°C, 143 ml d'une solution de n-Butyllithium (nBuLi) à 47,08 g du produit obtenu au stade A dans 375 ml de tétrahydrofuranne (THF), agite pendant 1 heure, puis ajoute 36,5 ml de triéthylborate. On agite 14 heures, en laissant remonter la température à 20°C, et on hydrolyse le milieu réactionnel au moyen d'une solution d'eau glacée contenant 45 ml d'acide sulfurique concentré, pendant 1 heure à 20°C. La phase aqueuse est extraite avec de l'acétate d'éthyle, les phases organiques sont lavées par de la soude 2N et la phase aqueuse est acidifiée à pH=1 à l'aide d'une solution d'acide chlorhydrique 1N afin de précipiter l'acide boronique. Après filtration et séchage du précipité on obtient 28,54 g du produit attendu.
Rf : 0,16 cyclohexane/acétate d'éthyle 7/3)
Spectre I.R. : (Nujol)

| | |
|---|---|
| Absorption générale région 0H/NH | 3650, 3615, 3510 et 3420 $cm^{-1}$ |
| Aromatique | 1605, 1570 et 1510 $cm^{-1}$ |
| B-O | 1410, 1340 $cm^{-1}$ |

**<u>PREPARATION 2</u> : Acide [4-[[(1,1-diméthyléthyl)diphénylsilyl] oxy]phényl]-boronique**

<u>Stade A</u> : 1-Bromo-4-[[(1,1-diméthyléthyl)diphénylsilyl]oxyl benzène

Sous atmosphère inerte et à température ambiante on ajoute à 80,89 g de parabromophénol, 400 ml de diméthylformamide, 31,18 g d'imidazole et 125,89 g de 1,1-diméthyl-éthyl-diphényl-chlorosilane puis on agite la solution obtenue pendant 2 heures. On verse dans 2 litres d'eau, observe une précipitation, solubilise le solide avec de l'acétate d'éthyle et extrait la phase aqueuse avec de l'acétate d'éthyle, sèche les phases organiques réunies et évapore sous pression réduite jusqu'à obtention d'une huile. On ajoute du pentane et observe une cristallisation. Après filtration et séchage du précipité on obtient 179,24 g du produit attendu. Rf : 0,53 (chromatographie sur couche mince, support : silice, éluant Cyclohexane/AcOEt 95/5).
Point de fusion : 56°C
RMN ($CDCl_3$, 300MHz)

| | |
|---|---|
| 1,09 s | SitBu |
| 6,63 m | $H_3$, $H_5$ |
| 7,17 m | $H_2$, $H_6$ |
| 7,69 dd | 4H pour SiΦ2 |
| 7,4 | 6H pour SiΦ2 |

<u>Stade B</u> : Acide [4-[[(1,1-diméthyléthyl)diphénylsilyl]oxy] phényl]-boronique

A une solution de 30 g du produit du stade précédent dans 100 ml de tétrahydrofuranne anhydre, on ajoute, goutte à goutte, à -78°C et sous gaz inerte, 60 ml d'une solution de n-butyl-lithium puis après agitation durant 30

minutes à - 78°C, 9,95 ml de triméthylborate. Après 2 heures trente d'agitation, la température du bain ayant évoluée jusqu'à 11,9°C, on additionne goutte à goutte 20 ml d'eau et agite pendant 72 heures à température ambiante. Après évaporation sous pression réduite du tétrahydrofuranne, on extrait la phase aqueuse à l'éther, sèche et concentre sous pression réduite jusqu'à obtention d'une huile (26,35 g) que l'on purifie par chromatographie filtrante sur silice avec le mélange hexane/acétate d'éthyle 1/1 pour obtenir 7,73 g du produit attendu sous forme de dimère.
IR ($CHCl_3$)

| | |
|---|---|
| O-Si | 915 et 1255 $cm^{-1}$ |
| B-O | 1350 et 1370 $cm^{-1}$ |
| Aromatiques | 1515, 1570 et 1602 $cm^{-1}$ |

RMN ($CDCl_3$)

| | |
|---|---|
| 1,11 | tBu |
| 6,81 et 7,88 | Ph-O |
| 7,3 à 7,5 (6H) et 7,72 (4H) | PhSi |

**<u>PRÉPARATION 3</u> : Acide-(4-méthoxyphényl)-boronique**

On ajoute goutte à goutte au reflux 100 ml d'une solution de 10 g de p-bromoanisole dans l'éther diéthylique anhydre à une suspension, sous gaz inerte, de 1,3 g de magnésium en tournure dans 5 ml d'éther diéthylique anhydre, et laisse le mélange au reflux pendant 2 heures. Le milieu réactionnel est ensuite versé dans une solution de 9,02 ml de triéthylborate dans 60 ml d'éther anhydre refroidie à -70°C. Après agitation durant 1 heure à -70°C, puis 1 heure à température ambiante, on verse la solution dans un mélange comprenant 11 ml d'acide sulfurique et 50 g de glace et d'eau et agite pendant 1 heure. On extrait la phase organique avec 100 ml d'une solution aqueuse saturée en bicarbonate de soude, réunit les phases aqueuses, les réacidifie avec de l'acide chlorhydrique 6N, extrait avec de l'éther, séche et évapore sous pression réduite. On obtient 3,9 g du produit attendu.
Spectre I.R. : (Nujol)
Absorption complexe région 0H/NH, 1609, 1573 et 1518 $cm^{-1}$
RMN (DMSO-d6, 300MHz)

| | |
|---|---|
| 3,76 s | OC<u>H</u>$_3$ |
| 6,88 d J=9Hz | $H_3$ et $H_5$ |
| 7,78 d J=9Hz | $H_2$ et $H_6$ |
| 7,86 | $B(OH)_2$ |

**<u>PRÉPARATION 4</u> : 4-(diméthylaminoéthoxy)phényl boronate.**

On refroidit à -78°C 2,5 g de 1-bromo 4-(diméthylamino) éthoxy benzène décrit dans le brevet RO 83118 dans 50 ml de tétrahydrofuranne puis ajoute en 5 minutes 7,86 ml de n-butyllithium. On agite 30 minutes à -78°C, ajoute en 10 minutes 1,35 ml de triméthyl borate, maintient 2 heures sous agitation à -78°C puis 3 heures à température ambiante. On ajoute goutte à goutte 3 ml d'eau et agite 72 heures à température ambiante. On évapore le solvant sous pression réduite, chromatographie le résidu sur silice (éluant THF) puis sur alumine neutre (éluant : $CH_2Cl_2$ puis $CH_2Cl_2$-MeOH 95-5). On obtient 810 mg de produit attendu.
Rf = 0,2 ($CH_2Cl_2$-MeOH 95-5).

**<u>EXEMPLE 1</u> : 5-[-4-[2-(diméthylamino)éthoxy]phényl]6-(4-hydroxyphényl)2-naphtalénol.**

<u>Stade A</u> : 1-[4-[2-(diméthylamino)éthoxy]phényl]6-méthoxy 2-naphtalénol.

a) 1-bromo 6-méthoxy 2-naphtalénol.
On dissout 1,05 g de 6-méthoxy 2-naphtalénol préparé comme àl'exemple 2 stade A dans 15 ml d'éthanol, ajoute 0,84 g de N-bromoacétamide et agite pendant 1 heure à température ambiante. On verse sur 800 ml d'eau, extrait au chlorure de méthylène, sèche, évapore le solvant sous pression réduite, purifie le résidu par chromatographie sur silice (éluant : cyclohexane-dichlorométhane 50-50). On recueille 0,75 g de produit attendu.
b) On agite au reflux pendant 3 heures, 2,5 g de produit obtenu comme au stade a) avec 2,45 g de 4-(diméthylamino-éthoxy) phényl boronate préparé comme indiqué à la préparation 4 dans 150 ml de dioxane en présence de 0,85 g de palladium tétrakis et 3,8 g de phosphate de potassium monohydrate. On évapore le solvant sous pression réduite, chromatographie le résidu sur silice (éluant : dichlorométhane-méthanol 95-5) et obtient 1,8 g de produit attendu.

Stade B : trifluorométhanesulfonate de 1-[4-(2-diméthylamino) éthoxy] phényl] 6-méthoxy 2-naphtalénol.

On mélange 1 g de produit obtenu au stade A dans 30 ml de pyridine puis ajoute 1,25 ml d'anhydride triflique. On chauffe 3 heures à 80°-90°C, verse dans une solution aqueuse saturée en bicarbonate de sodium. On extrait à l'acétate d'éthyle, sèche, évapore le solvant sous pression réduite, chromatographie le résidu sur silice (éluant : cyclohexane-acétate d'éthyle 7-3 puis dichlorométhane-méthanol 95-5). On obtient 0,72 g de produit attendu.
Rf = 0,3 (éluant : $CH_2Cl_2$-$CH_3OH$ 95-5).

Stade C : N,N-diméthyl 2-[4-[6-méthoxy 2-(4-méthoxyphényl) 1-naphthalényl]phénoxy]éthanamine.

On chauffe 3 heures à 120°C 0,64 g de triflate obtenu au stade B avec 274 mg d'acide 4-méthoxyphényl boronique préparé comme indiqué à la préparation 3 dans 30 ml de diméthylformamide en présence de 32 mg de tris (dibenzylidèneacétone) dipalladium, 505 mg de phosphate de potassium monohydrate, 177 mg de bromure de potassium et 73 mg de triphénylphosphine. On évapore le solvant sous pression réduite, chromatographie le résidu sur silice (éluant : dichlorométhane-méthanol 95-5 puis acétate d'éthyle-acétone-méthanol 80-10-10). On obtient 0,28 g de produit attendu.
Rf = 0,16 (AcOEt - $(CH_3)_2CO$ - $CH_3OH$ 80-10-10).
Spectre IR ($CHCl_3$)
aromatique : 1625, 1610, 1600, 1572, 1515, 1499 $cm^{-1}$.

Stade D : 5-[-4-[2-(diméthylamino)éthoxy]phényl]6-(4-hydroxyphényl)2-naphtalénol.

On chauffe 1 heure à 200°C 95 mg de produit obtenu comme au stade C avec 1,2 g de chlorhydrate de pyridinium. On reprend dans une solution aqueuse saturée en bicarbonate de sodium, extrait à l'acétate d'éthyle, sèche, évapore le solvant sous pression réduite, chromatographie le résidu sur silice (éluant : dichiorométhane-méthanol 90-10) et obtient 35 mg de produit attendu.
Rf = 0,07 (AcOEt - $(CH_3)_2$-CO - $CH_3OH$ 80-10-10).
Spectre IR (Nujol)
aromatique : 1620, 1608, 1508 $cm^{-1}$

**EXEMPLE 2 : 5-chloro-6-(4-hydroxyphenyl)-2-naphtalénol**

Stade A : protection du naphtol

2-hydroxy-6-méthoxy-naphtalène

On ajoute, sous atmosphère inerte, à 3,2 g de 2,6-dihydroxynaphtalène dans 20 ml de méthanol et 3 ml d'eau, 3,75 ml de sulfate de méthyle, 0,8 g de soude et on chauffe 15 heures à 40°C. Après avoir versé le milieu réactionnel dans 200 ml d'eau, on extrait à l'acétate d'éthyle, sèche, filtre et évapore sous pression réduite. Le produit brut est purifié par chromatographie 1,2 g de produit attendu ainsi que 1,6 g de l'analogue biprotégé.
Rf=0,24 (cyclohexane/acétate d'éthyle 80/20)
RMN ($CDCl_3$)

| | |
|---|---|
| 3,90 (s) | $OCH_3$ |
| 4,89 (sl) | OH |
| 7,58 d; 7,64 d | $H_4$; $H_8$ |

7,03 à 7,15 m 4H $H_1$, $H_3$, $H_5$, $H_7$

Stade B : Chloration

1-chloro-2-hydroxy-6-méthoxy-naphtalène

On ajoute sous atmosphère inerte 175 mg du produit préparé au stade précédent à une solution de chlorure de thionyle dans 5 ml de chloroforme à température ambiante et agite 1 heure. Après lavage à l'eau, séchage, filtration et évaporation sous pression réduite, on obtient 200 mg du produit attendu.
Rf=0,25 (cyclohexane/acétate d'éthyle 70/30)
$R_{MN}$ ($CDCl_3$)

| | |
|---|---|
| 3,91 s | $OCH_3$ |
| 5,71 s | OH |
| 7,59 d ; 7,97 d | $H_4$ ; $H_8$ |
| 7,23 m | $H_3$ |
| 7,11 d J=2,5Hz | $H_5$ |
| 7,23 m | $H_7$ |

Stade C : formation du triflate

1-chloro-2-trifluorométhylsulfonyloxy-6-méthoxy-naphtalène

A une solution, sous atmosphère inerte, de 600 mg de chloronaphtol obtenu au stade précédent dans 50 ml de pyridine, on ajoute, à 0°C, 0,75 ml d'anhydride triflique et agite 1,5 heures à température ambiante. On verse ensuite le milieu réactionnel dans 300 ml d'eau, extrait à l'acétate d'éthyle, sèche, filtre puis évapore sous pression réduite. On obtient 1 g du produit attendu que l'on utilise tel quel dans l'étape suivante.
Rf = 0,28 (cyclohexane/acétate d'éthyle 70/30)

Stade D : Couplage

1-chloro-2-(4-[[(1,1-diméthyléthyl)diphénylsilyl]oxy]phényl)-6-méthoxy-naphtalène

On mélange 8 heures, sous atmosphère inerte, au reflux, le mélange constitué de 1 g du triflate préparé au stade précédent, 1,5 g d'acide boronique obtenu à la préparation 2, 420 mg de bromure de potassium, 1,1 g de phosphate de potassium monohydrate, 100 mg de palladium tetrakis et 50 ml de dioxane. Après lavage à l'eau, séchage, filtration puis évaporation sous pression réduite, le produit brut est purifié par chromatographie en éluant avec le mélange cyclohexane/acétate d'éthyle 97/3. on obtient 1,4 g de produit attendu.
Rf = 0,55 (cyclohexane/acétate d'éthyle 90/10)

Stade E : Déprotection (désilylation)

1-chloro-2-(4-hydroxyphényl)-6-méthoxy-naphtalène

On agite 30 minutes, sous atmosphère inerte et à température ambiante 700 mg du produit obtenu au stade précédent dans 50 ml de tétrahydrofuranne et 1,5 ml de fluorure de tetrabutylammonium (1M/THF). Après évaporation sous pression réduite, le produit brut est purifié par chromatographie en éluant avec le mélange cyclohexane/acétate d'éthyle 90/10. On obtient 300 mg de produit attendu.
Rf= 0,45 (cyclohexane/acétate d'éthyle 70/30)
RMN ($CDCl_3$)

| | |
|---|---|
| 3,95 s | $OCH_3$ |
| 4,84 ml | OH |
| 7,16 d | $H_5$ |
| 7,29 dd | $H_7$ |
| 7,40 d, 7,68 dd, 8,29 d | $H_3$, $H_4$, $H_8$ |
| 6,93 ; 7,40 | H de Ph-OH |

Stade F : Déprotection : déméthylation

5-chloro-6-(4-hydroxyphényl)-2-naphtalénol

On mélange sous atmosphère inerte 300 mg du produit obtenu au stade précédent, 3 g de chlorhydrate de pyridinium, et on chauffe 2 heures à 200°C. Après avoir versé le milieu réactionnel dans 30 ml d'eau, on extrait à l'acétate d'éthyle, lave à l'eau, sèche, filtre et évapore sous pression réduite. Le produit brut est purifié par chromatographie en éluant avec le mélange dichlorométhane/éther 95/5. On obtient 120 mg de produit pur attendu
Rf=0,20 (cyclohexane/acétate d'éthyle 70/30)
F = 254°C
RMN ($CDCl_3$)

| | |
|---|---|
| 7,20 à 7,40 m,<br>7,71 dl, 8,13 dl | H du naphtyl |
| 6,87 ; 7,30 | H de Ph-OH |
| 9,57 s ; 10,00 s | OH (Ph-O<u>H</u>, Napht-O<u>H</u>) |

**EXEMPLE 3 : 1,5-dichloro-6-(4-hydroxyphényl)-2-naphtaléhol**

On opère de manière analogue à l'exemple 2 à partir de 100 mg de 1,5-dichloro-2-hydroxy-6-méthoxy-naphtalène (obtenu par action de 0,35 ml de chlorure de sulfuryle sur 350 mg du produit de l'exemple 2 stade A dans 20 ml de dichlorométhane avec un rendement de 100 %) et en utilisant l'acide boronique de la préparation 1 on obtient 15 mg de produit pur attendu. Rf= 0,60 (cyclohexane/acétate d'éthyle 50/50)
RMN ($CDCl_3$)

| | |
|---|---|
| 7,40 ; 7,53 d ; 8,04 d ; 8,30 d | H du naphtyl |
| 6,94 ; 7,430 | H de Ph-OH |
| 4,85 ml ; 5,92 ml | (Ph-O<u>H</u>, Napht-O<u>H</u>) |

**COMPOSITION PHARMACEUTIQUE**

On a préparé des comprimés répondant à la formule générale suivante :

- produit de l'exemple 2 50 mg
- Excipient (talc, amidon, stéarate de magnésium) qs pour un comprimé terminé à 120 mg

**ETUDE PHARMACOLOGIOUE DES PRODUITS DE L'INVENTION**

Récepteur estrogène humain (REH)

Un extrait cytosolique de cellules SF9 contenant le récepteur oestrogène humain recombinant est obtenu par surexpression dans un système cellules d'insectes-Baculovirus, selon la méthodologie générale décrite par N.R. WEBB et al. (Journal of Methods in Cell and Molecular Biology, (1990) Vol.2 n° 4, 173-188) et dont l'application est décrite à l'expression des récepteurs hormonaux humains, par exemple le récepteur glucocorticoide humain (G. SRINIVASAN et al. Molecular Endocrinology (1990) vol 4 n° 2 209-216).

On utilise le kit BaculoGold Transfection Kit (PharMingen, référence 21000K) pour générer le baculovirus recombinant contenant le fragment d'ADNc décrit dans le vecteur d'expression HEGO par L. TORA et al. (The EMBO Journal (1989) vol. 8 n° 7 1981-1986), comprenant la région codante pour le récepteur oestrogène humain de type sauvage avec une glycine en position 400.

Le virus recombinant ainsi obtenu est utilisé pour exprimer le récepteur estrogène dans les cellules d'insectes SF9 (ATCC CRL1711), selon la méthodologie connue citée précédemment.

$2*10^7$ cellules SF9 sont cultivées dans un flacon "Falcon" de 175 $cm^2$ dans le milieu TNM-FH "SIGMA" supplémenté avec 10 % de sérum de veau foecal (SVF) et avec 50 micro-gramme/ml de gentamycine. Après infection puis incubation à 27°C pendant 40 à 42 heures, les cellules sont lysées dans 1 ml de tampon de lyse (Tris 20 mM-HCl pH8, EDTA 0,5 mM, DTT 2 mM, Glycérol 20 %, KCl 400 mM) par un cycle de congélation-décongélation que l'on répète encore deux fois. Le surnageant, contenant le récepteur estrogène humain recombinant est conservé dans l'azote

liquide par dose de 0,5 ml.

Le surnageant est incubé à 0°C pendant 24 heures avec une concentration constante (T) d'oestradiol tritié en présence de concentrations croissantes soit d'estradiol froid (0 - $1000x10^{-9}M$) , soit du produit froid à tester (0 - $25000x10^{-9}M$). La concentration d'estradiol tritié liée (B) est ensuite mesurée dans chaque incubat par la technique d'adsorption au charbon dextran.

<u>Calcul de l'affinité relative de liaison</u> (ARL) :

On trace les deux courbes suivantes : le pourcentage de l'hormone tritiée liée 100xB/B0 en fonction du logarithme de la concentration de l'hormone de référence froide ou en fonction du logarithme de la concentration du produit froid testé.

On détermine la droite d'équation

$$I_{50} = 100\ (B0/B0 + Bmin/B0)/2 = 100(1+Bmin/B0) = 50(1+Bmin/B0)$$

B0 = Concentration de l'hormone tritiée liée en l'absence de tout produit froid.
B = Concentration de l'hormone tritiée liée en présence d'une concentration X de produit froid.
Bmin = Concentration de l'hormone tritiée liée pour une incubation de cette hormone tritiée à la concentration (T) en présence d'un grand excès d'hormone froide de référence ($1000x10^{-9}M$) pour récepteur humain.

Les intersections de la droite $I_{50}$ et des courbes permettant d'évaluer les concentrations de l'hormone de référence froide (CH) et du produit froid testé (CX) qui inhibent de 50 % la liaison de l'hormone tritiée sur le récepteur.

L'affinité relative de liaison (ARL) du produit testé est déterminée par l'équation ARL=100(CH)/(CX).

Les résultats obtenus sont les suivants :

| Exemples | REH estradiol = 100 24 h |
|---|---|
| 1 | 28 |
| 2 | 49 |
| 3 | 5 |

**Conclusion :**

Bien que ces produits aient une affinité de 2 à 20 fois plus faible que celle de l'estradiol, la fixation sur récepteur estrogène est un fait nouveau pour cette famille de produits.

## Revendications

1. Les composés de formule générale (I) :

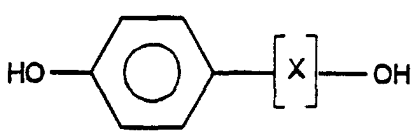

(I)

dans laquelle [X] représente les carbocycles aromatiques suivants :

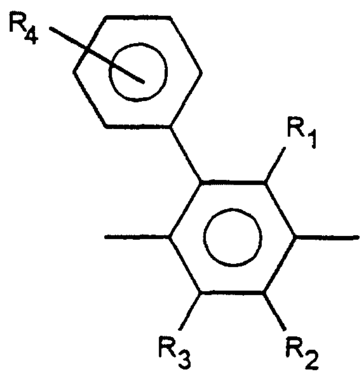

(A)

ou

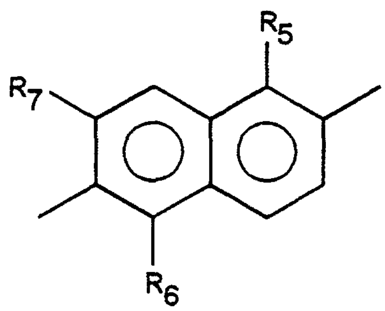

(B)

et dans lesquels $R_1$ représente un radical alkyle renfermant de 1 à 4 atomes de carbone ou un atome d'hydrogène, $R_2$ représente un radical alkyle renfermant de 1 à 4 atomes de carbone ou un atome d'hydrogène, $R_3$ représente un atome d'hydrogène, un atome d'halogène, un radical alkyle renfermant de 1 à 4 atomes de carbone ou un radical alkoxy renfermant de 1 à 4 atomes de carbone, $R_4$ en position para ou méta représente un atome d'hydrogène, un atome d'halogène, un radical hydroxyle, un radical alkyle, alkényle ou alkynyle renfermant au plus 4 atomes de carbone, un radical alkoxy, alkylthio, dans lesquels alkyle renferme de 1 à 4 atomes de carbone, un groupement $-NR_AR_B$ dans lequel $R_A$ et $R_B$ identiques ou différents représentent un atome d'hydrogène, un radical alkyle renfermant de 1 à 4 atomes de carbone ou forment ensemble avec l'azote auquel ils sont liés un hétérocycle saturé à 5 ou 6 chaînons renfermant éventuellement un second hétéroatome choisi parmi l'azote, l'oxygène et le soufre, ce groupement $-NR_AR_B$ étant éventuellement sous forme oxydée, un groupement de formule générale $-O-(CH_2)_n-NR_AR_B$ dans laquelle n est un entier qui varie de 2 à 7 et dans laquelle $-NR_AR_B$ est tel que défini précédemment, $R_5$ représente un atome d'hydrogène ou un atome d'halogène, $R_6$ et $R_7$ identiques ou différents représentent un atome d'hydrogène, un atome d'halogène, un radical alkyle renfermant de 1 à 4 atomes de carbone ou un radical phényle éventuellement substitué en position méta ou para par un radical $R_4$ tel que défini précédemment ainsi que les sels d'addition avec les acides ou les bases, à l'exception des composés de formule (I) dans laquelle [X] représente le groupe (A) dans lequel $R_1$, $R_2$, $R_3$ sont des atomes d'hydrogène et $R_4$ représente un radical hydroxyle et ceux dans laquelle [X] représente le groupe (B) dans lequel $R_5$, $R_6$ et $R_7$ sont des atomes d'hydrogène ou bien $R_5$ et $R_6$ sont des atomes d'hydrogène et $R_7$ représente un radical alkyle renfermant de 1 à 4 atomes de carbone.

**2.** Les composés de formule générale (I) telle que définie à la revendication 1 dans laquelle [X] est le carbocycle aromatique de formule générale (A).

**3.** Les composés de formule générale (I) telle que définie à la revendication 1 dans laquelle [X] est le carbocycle aromatique de formule générale (B).

**4.** Les composés de formule générale (I) telle que définie à la revendication 1 ou 2, répondant à la formule générale (I') :

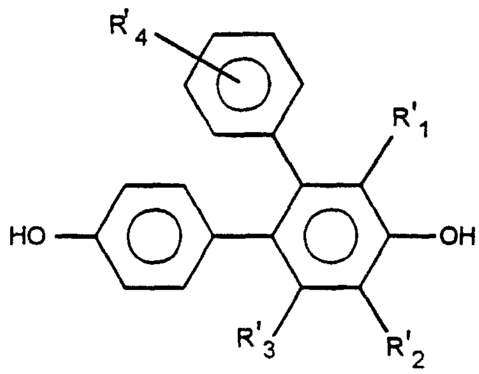

(I')

dans laquelle R'$_1$, R'$_2$ et R'$_3$ représentent un atome d'hydrogène ou un radical alkyle renfermant de 1 à 4 atomes de carbone, R'$_4$ en position méta ou para représente un atome d'hydrogène, un atome d'halogène, un radical hydroxyle, un radical alkyle, renfermant de 1 à 4 atomes de carbone, un groupement $-NR_AR_B$ ou un groupement $-O-(CH_2)_n-NR_AR_B$, n, $R_A$ et $R_B$ étant tels que définis à la revendication 1, ainsi que les sels d'addition avec les acides ou les bases.

**5.** Les composés répondant à la formule générale (I') telle que définie à la revendication 4 dans laquelle R'$_1$, R'$_2$ et R'$_3$ sont des atomes d'hydrogène.

**6.** Les composés de formule générale (I) telle que définie à la revendication 1 ou 3, dans laquelle $R_6$ représente un atome d'halogène ou un groupement

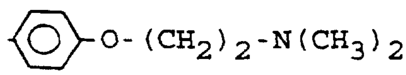

et $R_7$ représente un atome d'hydrogène.

**7.** Les composés de formule générale (I) telle que définie aux revendications 1 à 6 suivants :

- 5-[4-[2-(diméthylamino)éthoxy]phényl]6-(4-hydroxyphényl) 2-naphtalénol,
- 1,5-dichloro-6-(4-hydroxyphényl)-2-naphtalénol,
- 5-chloro-6-(4-hydroxyphényl)-2-naphtalénol.

**8.** Un procédé de préparation des produits de formule (I) telle que définie à la revendication 1, **caractérisé en ce que** l'on soumet un produit de formule (II) :

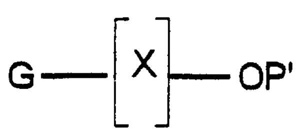

(II)

dans laquelle [X] est tel que défini à la revendication 1, P' représente un groupement protecteur, et G représente un atome d'halogène ou un groupement $OSO_2CF_3$ à l'action, en présence d'un catalyseur, d'un produit de formule (III) :

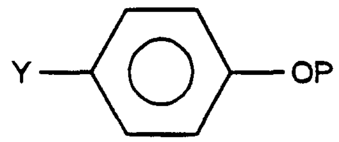

(III)

dans laquelle Y représente un atome d'halogène, un groupement $B(OH)_2$ ou un groupement $Sn(R)_3$, dans lequel R représente un groupement alkyle renfermant de 1 à 8 atomes de carbone et P représente un groupement protecteur, pour obtenir un produit de formule (IV) :

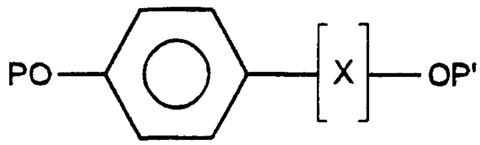

(IV)

dans laquelle P, P' et [X] ont la même signification que précédemment, produit de formule (IV) que l'on soumet à une ou plusieurs réactions de déprotection afin d'obtenir le produit attendu de formule (I) que si désiré, l'on soumet à l'action d'un acide ou d'une base pour obtenir le sel correspondant.

**9.** Un procédé de préparation des produits de formule (I) telle que définie à la revendication 2, **caractérisé en ce que** l'on soumet un produit de formule (V) :

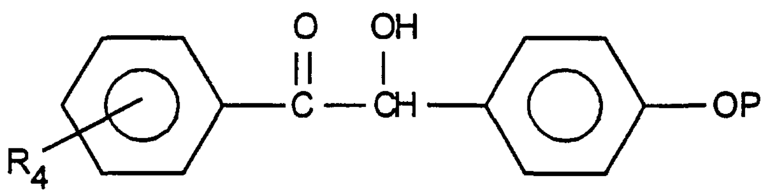

(V)

dans laquelle $R_4$ est tel que défini à la revendication 1, et dans laquelle P représente un groupement protecteur, à l'action de la méthylvinylcétone de formule générale (VI) :

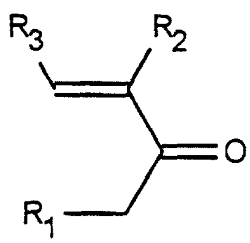

(VI)

dans laquelle $R_1$, $R_2$ et $R_3$ sont tels que définis à la revendication 1, pour obtenir le produit de formule (VII) :

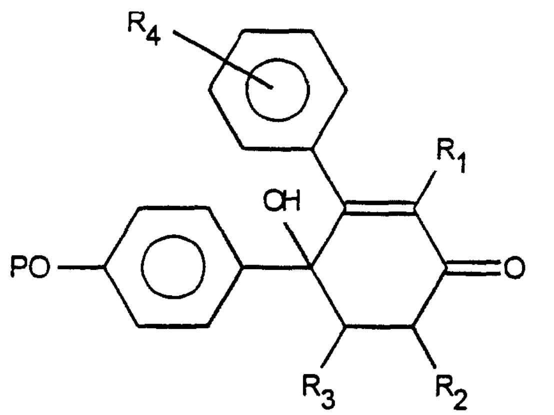

(VII)

dans laquelle $R_1$, $R_2$, $R_3$, $R_4$ et P sont tels que définis précédemment,
que l'on soumet à l'action d'un réactif de déshydratation et d'aromatisation afin d'obtenir le produit de formule (VIII) :

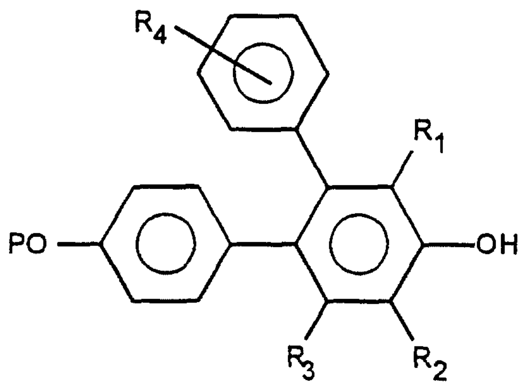

(VIII)

dans laquelle $R_1$, $R_2$, $R_3$, $R_4$ et P sont tels que définis à la revendication 1,
que l'on soumet à l'action d'un réactif de déprotection afin d'obtenir les produits attendus de formule (I) que si désiré, l'on soumet à l'action d'un acide ou d'une base pour obtenir le sel correspondant.

**10.** A titre de médicaments, les produits de formule générale (I) telle que définie à la revendication 1, ainsi que leurs sels d'addition avec les acides ou les bases pharmaceutiquement acceptables.

**11.** A titre de médicaments les produits de formule générale (I) telle que définie à l'une quelconque des revendications 2 à 6, ainsi que leurs sels d'addition avec les acides ou les bases pharmaceutiquement acceptables.

**12.** A titre de médicaments les produits de formule générale (I) tel que définis à la revendication 7.

**13.** Compositions pharmaceutiques renfermant comme principe actif l'un au moins des médicaments tels que définis à l'une quelconque des revendications 10 à 12.

**14.** A titre de produits industriels nouveaux les produits de formule générale (IV), (VII) et (VIII) telles que définies précédemment, à l'exception des produits de formule (VII) et (VIII) dans laquelle $R_4$ est un radical méthoxy, P est un radical méthyle et $R_1$, $R_2$ et $R_3$ sont des atomes d'hydrogène, et à l'exception des produits de formule (IV) dans laquelle P et P' sont des radicaux méthyle ou acyle et X représente le groupe B dans lequel $R_5$, $R_6$ et $R_7$ sont des atomes d'hydrogène.

**Claims**

**1.** The compounds of general formula (I):

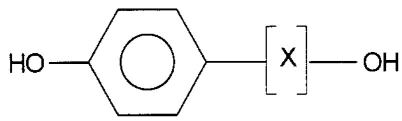

(I)

in which [X] represents the following aromatic carbocycles:

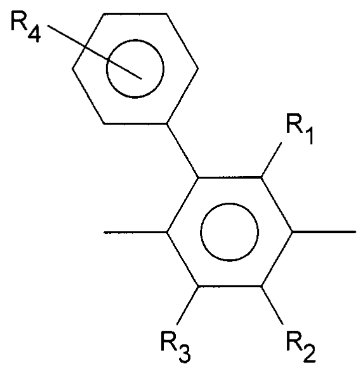

(A)

or

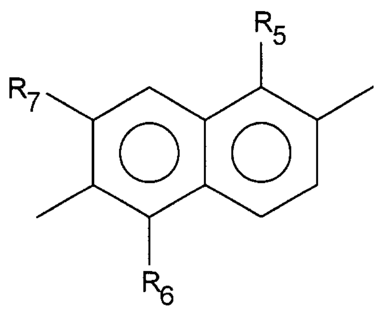

(B)

and in which $R_1$ represents an alkyl radical containing 1 to 4 carbon atoms or a hydrogen atom, $R_2$ represents an alkyl radical containing 1 to 4 carbon atoms or a hydrogen atom, $R_3$ represents a hydrogen atom, a halogen atom, an alkyl radical containing 1 to 4 carbon atoms or an alkoxy radical containing 1 to 4 carbon atoms, $R_4$ in para or meta position represents a hydrogen atom, a halogen atom, a hydroxyl radical, an alkyl, alkenyl or alkynyl radical containing at most 4 carbon atoms, an alkoxy, alkylthio radical, in which alkyl contains 1 to 4 carbon atoms, an $-NR_AR_B$ group in which $R_A$ and $R_B$ identical or different represent a hydrogen atom, an alkyl radical containing 1 to 4 carbon atoms or together with the nitrogen to which they are linked form a saturated heterocycle with 5 or 6 members optionally containing a second heteroatom chosen from nitrogen, oxygen and sulphur, this $-NR_AR_B$ group being optionally in oxidized form, a group of general formula $-O-(CH_2)_n-NR_AR_B$ in which n is an integer which varies from 2 to 7 and in which $-NR_AR_B$ is as defined previously, $R_5$ represents a hydrogen atom or a halogen atom, $R_6$ and $R_7$ identical or different represent a hydrogen atom, a halogen atom, an alkyl radical containing 1 to 4 carbon atoms or a phenyl radical optionally substituted in meta or para position by an $R_4$ radical as defined previously as well as the addition salts with acids or bases, with the exception of the compounds of formula (I) in which [X] represents the group (A) in which $R_1$, $R_2$, $R_3$ are hydrogen atoms and $R_4$ represents a hydroxyl radical and those in which [X] represents the group (B) in which $R_5$, $R_6$ and $R_7$ are hydrogen atoms or $R_5$ and $R_6$ are hydrogen atoms and $R_7$ represents an alkyl radical containing 1 to 4 carbon atoms.

**2.** The compounds of general formula (I) as defined in claim 1 in which [X] is the aromatic carbocycle of general formula (A).

**3.** The compounds of general formula (I) as defined in claim 1 in which [X] is the aromatic carbocycle of general formula (B).

**4.** The compounds of general formula (I) as defined in claim 1 or 2, corresponding to general formula (I'):

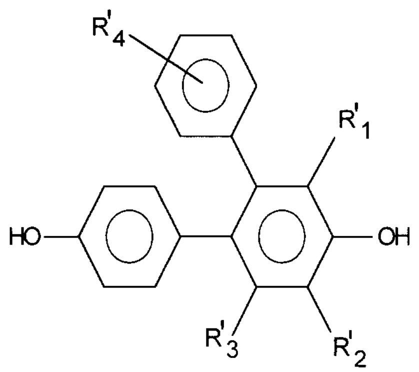

(I')

in which $R'_1$, $R'_2$ and $R'_3$ represent a hydrogen atom or an alkyl radical containing 1 to 4 carbon atoms, $R'_4$ in meta or para position represents a hydrogen atom, a halogen atom, a hydroxyl radical, an alkyl radical containing 1 to 4 carbon atoms, an $NR_AR_B$ group or an $O-(CH_2)_n-NR_AR_B$ group, n, $R_A$ and $R_B$ being as defined in claim 1, as well as the addition salts with acids or bases.

**5.** The compounds corresponding to general formula (I') as defined in claim 4 in which $R'_1$, $R'_2$ and $R'_3$ are hydrogen atoms.

**6.** The compounds of general formula (I) as defined in claim 1 or 3, in which $R_6$ represents a halogen atom or a $-O-(CH_2)_2-N(CH_3)_2$ group and $R_7$ represents a hydrogen atom.

**7.** The following compounds of general formula (I) as defined in claims 1 to 6:

- 5-[4-[2-(dimethylamino)ethoxy]phenyl]6-(4-hydroxyphenyl) 2-naphthalenol,
- 1,5-dichloro-6-(4-hydroxyphenyl)-2-naphthalenol,
- 5-chloro-6-(4-hydroxyphenyl)-2-naphthalenol.

**8.** A process for the preparation of the products of formula (I) as defined in claim 1, **characterized in that** a product of formula (II):

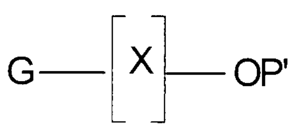

(II)

in which [X] is as defined in claim 1, P' represents a protective group and G represents a halogen atom or an $OSO_2CF_3$ group is subjected to the action, in the presence of a catalyst, of a product of formula (III):

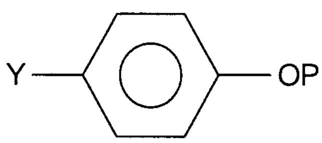

(III)

in which Y represents a halogen atom, a $B(OH)_2$ group or an $Sn(R)_3$ group, in which R represents an alkyl group

containing 1 to 8 carbon atoms and P represents a protective group, in order to obtain a product of formula (IV):

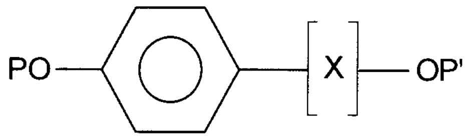

(IV)

in which P, P' and [X] have the same meaning as previously, which product of formula (IV) is subjected to one or more deprotection reactions in order to obtain the expected product of formula (I) which, if desired, is subjected to the action of an acid or base in order to obtain the corresponding salt.

**9.** A process for the preparation of the products of formula (I) as defined in claim 2, **characterized in that** a product of formula (V):

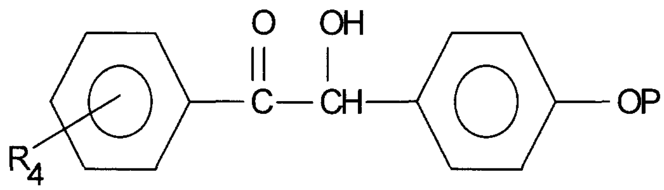

(V)

in which $R_4$ is as defined in claim 1, and in which P represents a protective group, is subjected to the action of the methylvinylketone of general formula (VI):

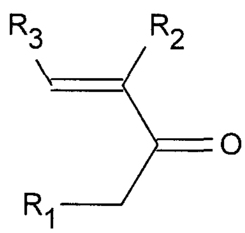

(VI)

in which $R_1$, $R_2$ and $R_3$ are as defined in claim 1, in order to obtain the product of formula (VII):

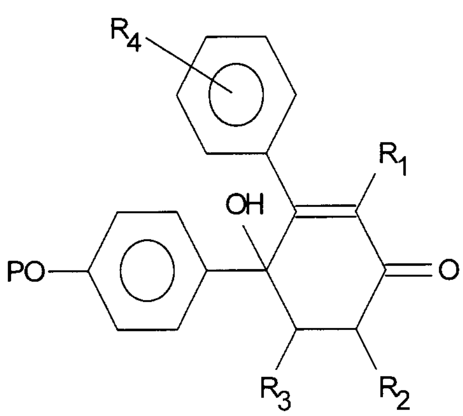

(VII)

in which $R_1$ , $R_2$, $R_3$, $R_4$ and P are as defined previously, which is subjected to the action of a dehydration and aromatisation reaction in order to obtain the product of formula (VIII):

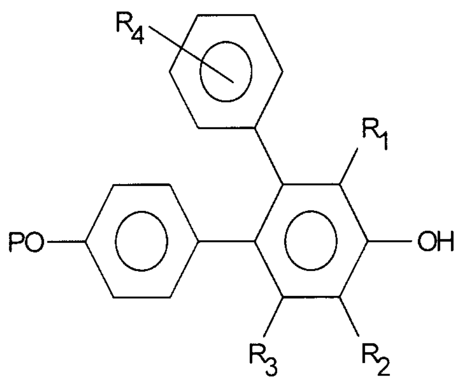

(VIII)

in which $R_1$, $R_2$, $R_3$, $R_4$ and P are as defined in claim 1, which is subjected to the action of a deprotection reagent in order to obtain the expected products of formula (I) which if desired, are subjected to the action of an acid or base in order to obtain the corresponding salt.

**10.** As medicaments, the products of general formula (I) as defined in claim 1, as well as their addition salts with pharmaceutically acceptable acids or bases.

**11.** As medicaments the products of general formula (I) as defined in any one of claims 2 to 6, as well as their addition salts with pharmaceutically acceptable acids or bases.

**12.** As medicaments the products of general formula (I) as defined in claim 7.

**13.** Pharmaceutical compositions containing as active ingredient at least one of the medicaments as defined in any one of claims 10 to 12.

**14.** As new industrial products the products of general formula (IV), (VII) and (VIII) as defined previously, with the exception of the products of formula (VII) and (VIII) in which $R_4$ is a methoxy radical, P is a methyl radical and $R_1$, $R_2$ and $R_3$ are hydrogen atoms, and with the exception of the products of formula (IV) in which P and P' are methyl or acyl radicals and X represents the group B in which $R_5$, $R_6$ and $R_7$ are hydrogen atoms.

**Patentansprüche**

**1.** Verbindungen der allgemeinen Formel (I)

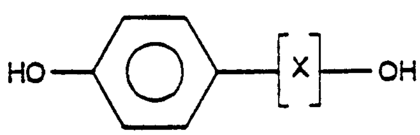

(I)

worin [X] die folgenden aromatischen Carbocyclen darstellt:

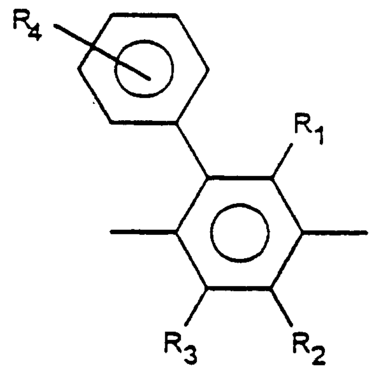

(A)

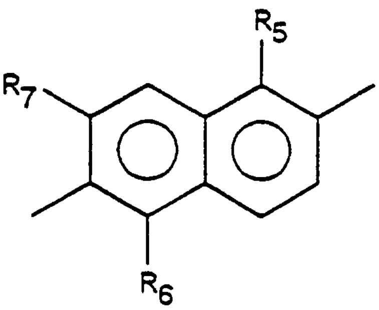

(B)

worin $R_1$ eine Alkyl-Gruppe, die 1 bis 4 Kohlenstoffatome umfaßt, oder ein Wasserstoffatom darstellt; $R_2$ eine Alkyl-Gruppe, die 1 bis 4 Kohlenstoffatome umfaßt, oder ein Wasserstoffatom darstellt; $R_3$ ein Wasserstoffatom, ein Halogenatom, eine Alkyl-Gruppe, die 1 bis 4 Kohlenstoffatome umfaßt, oder eine Alkoxy-Gruppe, die 1 bis 4 Kohlenstoffatome umfaßt, darstellt; $R_4$ in paraoder meta-Stellung ein Wasserstoffatom, ein Halogenatom, eine Hydroxyl-Gruppe, eine Alkyl-, Alkenyl- oder Alkinyl-Gruppe, die höchstens 4 Kohlenstoffatome umfaßt, eine Alkoxy-, eine Alkylthio-Gruppe, in der Alkyl 1 bis 4 Kohlenstoffatome umfaßt, eine Gruppierung $-NR_AR_B$, worin $R_A$ und $R_B$, die gleich oder unterschiedlich sind, ein Wasserstoffatom, eine Alkyl-Gruppe, die 1 bis 4 Kohlenstoffatome umfassen, oder zusammen mit dem Stickstoff, an den sie gebunden sind, einen gesättigten 5- oder 6-gliedrigen Heterocyclus bilden, der gegebenenfalls ein zweites Heteroatom, ausgewählt aus Stickstoff, Sauerstoff und Schwefel, umfaßt, wobei diese Gruppierung $-NR_AR_B$ gegebenenfalls in oxidierter Form vorliegt, eine Gruppierung der allgemeinen Formel $-O-(CH_2)_n-NR_AR_B$, worin n eine ganze Zahl ist, die zwischen 2 und 7 variiert, $-NR_AR_B$ wie vorstehend definiert ist, darstellt; $R_5$ ein Wasserstoffatom oder ein Halogenatom darstellt; $R_6$ und $R_7$, die gleich oder unterschiedlich sind, ein Wasserstoffatom, ein Halogenatom, eine Alkyl-Gruppe, die 1 bis 4 Kohlenstoffatome umfaßt, oder eine Phenyl-Gruppe, die gegebenenfalls in meta-Stellung oder para-Stellung durch eine Gruppe $R_4$, wie sie vorstehend definiert ist, substituiert ist, darstellen;
sowie die Additionssalze mit Säuren und Basen, ausgenommen Verbindungen der Formel (I), in der [X] die Gruppe (A) darstellt, worin $R_1$, $R_2$, $R_3$ Wasserstoffatome sind und $R_4$ eine Hydroxyl-Gruppe darstellt, und die, in denen [X] die Gruppe (B) darstellt, worin $R_5$, $R_6$ und $R_7$ Wasserstoffatome sind oder aber $R_5$ und $R_6$ Wasserstoffatome sind und $R_7$ eine Alkyl-Gruppe, die 1 bis 4 Kohlenstoffatome umfaßt, darstellt.

**2.** Verbindungen der allgemeinen Formel (I) nach Anspruch 1, wobei [X] der aromatische Carbocyclus der allgemeinen Formel (A) ist.

**3.** Verbindungen der allgemeinen Formel (I) nach Anspruch 1, wobei [X] der aromatische Carbocyclus der allgemeinen Formel (B) ist.

**4.** Verbindungen der allgemeinen Formel (I) nach Anspruch 1 oder Anspruch 2, die der allgemeinen Formel (I') entsprechen:

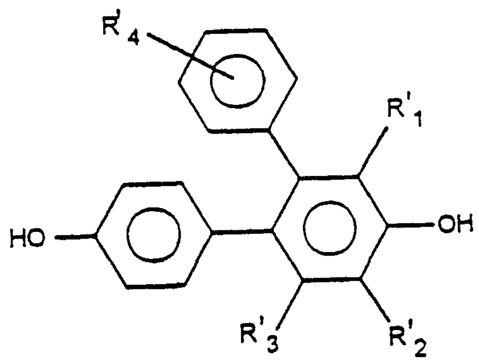

(I')

worin $R'_1$, $R'_2$ und $R'_3$ ein Wasserstoffatom oder eine Alkyl-Gruppe, die 1 bis 4 Kohlenstoffatome umfaßt, darstellen; $R'_4$ in meta- oder para-Stellung ein Wasserstoffatom, ein Halogenatom, eine Hydroxyl-Gruppe, eine Alkyl-Gruppe, die 1 bis 4 Kohlenstoffatome umfaßt, eine Gruppierung $-NR_AR_B$ oder eine Gruppierung $-O-(CH_2)_n-NR_AR_B$, worin n, $R_A$ und $R_B$ wie in Anspruch 1 definiert sind, darstellt, sowie die Additionssalze mit Säuren oder Basen.

**5.** Verbindungen der allgemeinen Formel (I') nach Anspruch 4, in der $R'_1$, $R'_2$ und $R'_3$ Wasserstoffatome sind.

**6.** Verbindungen der allgemeinen Formel (I) nach Anspruch 1 oder Anspruch 3, worin $R_6$ ein Wasserstoffatom oder eine Gruppierung

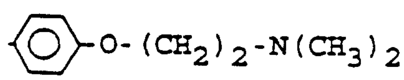

darstellt und $R_7$ ein Wasserstoffatom darstellt.

**7.** Folgende Verbindungen der allgemeinen Formel (I) nach den Ansprüchen 1 bis 6:

- 5-[4-[2-(Dimethylamino)ethoxy]phenyl]-6-(4-hydroxyphenyl)-2-naphthalinol,
- 1,5-Dichlor-6-(4-hydroxyphenyl)-2-naphthalinol,
- 5-Chlor-6-(4-hydroxyphenyl)-2-naphthalinol.

**8.** Verfahren zur Herstellung der Produkte der Formel (I) nach Anspruch 1, **dadurch gekennzeichnet, daß** man ein Produkt der Formel (II):

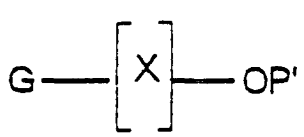

(II)

in der [X] wie in Anspruch 1 definiert ist, P' eine Schutzgruppe darstellt und G ein Halogenatom oder eine Gruppierung $OSO_2CF_3$ darstellt, in Gegenwart eines Katalysators der Wirkung eines Produktes der Formel (III):

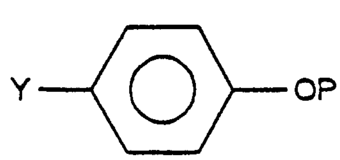

(III)

in der Y ein Halogenatom, eine Gruppierung $B(OH)_2$ oder eine Gruppierung $Sn(R)_3$, worin R eine Alkyl-Gruppierung, die 1 bis 8 Kohlenstoffatome umfaßt, darstellt und P eine Schutzgruppe darstellt, unterwirft, um ein Produkt der Formel (IV) zu erhalten:

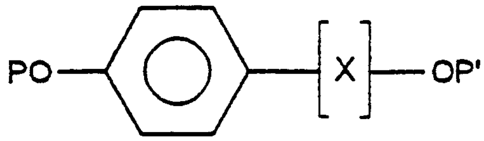

(IV)

in der P, P' und [X] dieselbe Bedeutung wie vorstehend haben; man das Produkt der Formel (IV) einer Reaktion oder mehreren Reaktionen zur Schutzgruppenentfernung unterwirft, um das erwartete Produkt der Formel (I) zu erhalten, das man, wenn gewünscht, der Wirkung einer Säure oder Base unterwirft, um das entsprechende Salz zu erhalten.

**9.** Verfahren zur Herstellung der Produkte der Formel (I) nach Anspruch 2, **dadurch gekennzeichnet, daß** man ein Produkt der Formel (V):

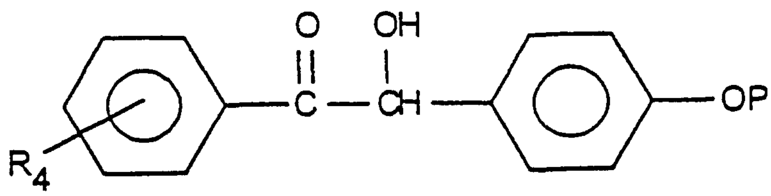

(V)

in der $R_4$ wie in Anspruch 1 definiert und in der P eine Schutzgruppe darstellt, der Wirkung von Methylvinylketon der allgemeinen Formel (VI):

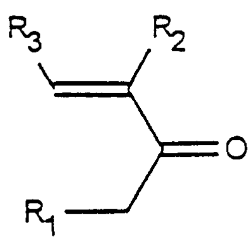

(VI.)

worin $R_1$, $R_2$ und $R_3$ wie in Anspruch 1 definiert sind, unterwirft, um das Produkt der Formel (VII):

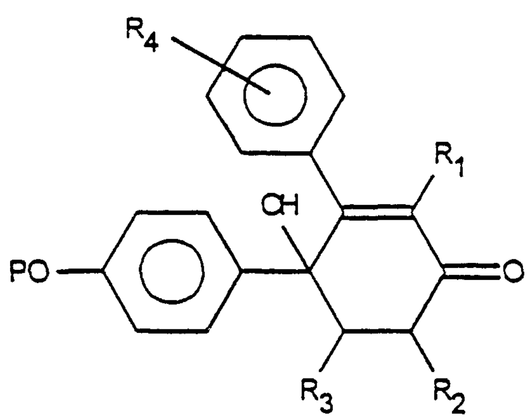

(VII)

in der $R_1$, $R_2$, $R_3$, $R_4$ und P wie vorher definiert sind, zu erhalten,
das man der Wirkung eines Dehydratisierungs- und Aromatisierungs-Reagenzes unterwirft, um das Produkt der Formel (VIII) zu erhalten:

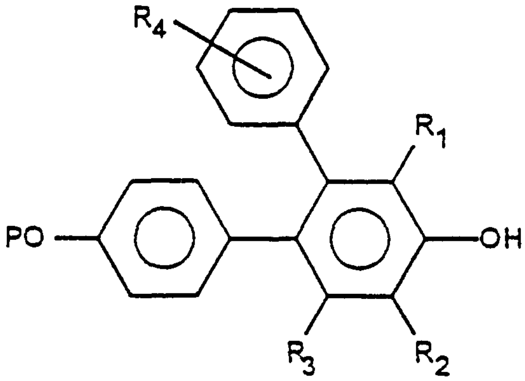

(VIII)

in der $R_1$, $R_2$, $R_3$, $R_4$ und P wie in Anspruch 1 definiert sind,
das man der Wirkung eines Reagenzes zur Schutzgruppenentfernung unterwirft, um die erwarteten Produkte der Formel (I) zu erhalten, die man, wenn gewünscht, der Wirkung einer Säure oder Base unterwirft, um das entsprechende Salz zu erhalten.

**10.** Produkte der allgemeinen Formel (I) nach Anspruch 1 sowie ihre Additionssalze mit pharmazeutisch annehmbaren Säuren und Basen als Arzneimittel.

**11.** Produkte der allgemeinen Formel (I) nach einem der Ansprüche 2 bis 6 sowie ihre Additionssalze mit pharmazeutisch annehmbaren Säuren oder Basen als Arzneimittel.

**12.** Produkte der allgemeinen Formel (I) nach Anspruch 7 als Arzneimittel.

**13.** Pharmazeutische Zusammensetzungen, die als Wirksubstanz mindestens eins der Arzneimittel nach einem der Ansprüche 10 bis 12 umfassen.

**14.** Produkte der allgemeinen Formeln (IV), (VII) und (VIII), wie sie vorstehend definiert sind, ausgenommen Produkte der Formel (VII) und (VIII), worin $R_4$ eine Methoxy-Gruppe, P eine Methyl-Gruppe ist und $R_1$, $R_2$ und $R_3$ Wasserstoffatome sind, und ausgenommen Produkte der Formel (IV), worin P und P' Methyl- oder Acyl-Gruppen sind und X die Gruppe B, in der $R_5$, $R_6$ und $R_7$ Wasserstoffatome sind, darstellt, als neue neue industrielle Produkte.